Europäisches Patentamt

**European Patent Office**

**Office européen des brevets**

(19)

(11) **EP 0 928 156 B1**

(12) **EUROPÄISCHE PATENTSCHRIFT**

(45) Veröffentlichungstag und Bekanntmachung des Hinweises auf die Patenterteilung:
**06.03.2002 Patentblatt 2002/10**

(21) Anmeldenummer: **97940146.0**

(22) Anmeldetag: **29.08.1997**

(51) Int Cl.[7]: **A61B 5/026**, A61B 5/0275

(86) Internationale Anmeldenummer:
**PCT/EP97/04725**

(87) Internationale Veröffentlichungsnummer:
**WO 98/08434 (05.03.1998 Gazette 1998/09)**

(54) **ANORDNUNG ZUR NICHT INVASIVEN BESTIMMUNG DES ZEREBRALEN BLUTFLUSSES MITTELS NAH-INFRAROT-SPEKTROSKOPIE**

DEVICE FOR NON-INVASIVELY DETERMINING CEREBRAL BLOOD FLOW BY NEAR-INFRARED SPECTROSCOPY

DISPOSITIF POUR LA DETERMINATION NON INVASIVE DU FLUX SANGUIN CEREBRAL PAR SPECTROSCOPIE DANS L'INFRAROUGE PROCHE

(84) Benannte Vertragsstaaten:
**DE ES FR GB IT**

(30) Priorität: **29.08.1996 DE 19635038**

(43) Veröffentlichungstag der Anmeldung:
**14.07.1999 Patentblatt 1999/28**

(73) Patentinhaber: **Pulsion Medical Systems AG
81829 München (DE)**

(72) Erfinder:
• **FEIFFER, Ulrich, J.
D-81667 München (DE)**
• **KÜBLER, Wolfgang
D-81545 München (DE)**
• **GOETZ, Alwin, E.
D-81477 München (DE)**

(74) Vertreter: **Kehl, Günther, Dipl.-Phys.
Patentanwaltskanzlei Günther Kehl
Friedrich-Herschel-Strasse 9
81679 München (DE)**

(56) Entgegenhaltungen:
**EP-A- 0 502 270          EP-A- 0 615 723
WO-A-96/16594          DE-A- 4 130 931
US-A- 4 805 623**

Bemerkungen:
Die Akte enthält technische Angaben, die nach dem Eingang der Anmeldung eingereicht wurden und die nicht in dieser Patentschrift enthalten sind.

## Beschreibung

Die vorliegende Erfindung betrifft eine Anordnung zur Messung des Blutflusses durch ein Organ mittels Nah-Infrarot-Spektroskopie unter intravenöser Applikation eines Tracerfarbstoffes.

Bekannt ist eine 1988 von Edwards et al. (Edwards, A. D. et al.: The Lancet 2 (1988), 770-771) entwickelte, nicht invasive Methode zur Messung des zerebralen Blutflusses mittels Nah-Infrarot-Spektroskopie (NIRS) .

Die Nah-Infrarot-Spektroskopie (NIRS), 1977 erstmals von Jöbsis beschrieben (Jöbsis, F. F.: Science 198 (1977), 1264-1267), stellt eine relativ neue Methode dar, die die nicht invasive Erfassung von Veränderungen der Konzentration von Chromophoren mit Absorptionseigenschaften im nah-infraroten Spektrum im Gewebe ermöglicht. Das von einem Laser an der Körperoberfläche emittierte Licht im nah-infraroten Spektrum ($\lambda$ = 700 - 1000 nm) kann im Gegensatz zu sichtbarem Licht körpereigenes Gewebe bis zu einer Tiefe von 6 cm durchdringen (van der Zee, P. et al.: Adv. Exp. Med. Biol. 316 (1992), 143-153). Ein Teil des Lichtes wird dabei gestreut und reflektiert und kann wiederum an der Körperoberfläche über einen Photomultiplier detektiert werden. Chromophore im Gewebe mit Absorptionseigenschaften im Bereich des emittierten Lichtes vermögen, in Abhängigkeit von ihrer Konzentration, das einfallende Licht zu absorbieren und so die emittierte Lichtmenge zu vermindern. Entsprechend dem Lambert-Beer'schen Gesetz kann aus der Lichtabsorption, d. h. der optischen Dichte des Gewebes, auf die Konzentration des Chromophors im Gewebe rückgeschlossen werden.

Die von Edwards et al. entwickelte Methode verwendet $HbO_2$, dessen Konzentration im Blut durch plötzliche Erhöhung des Sauerstoffgehalts der inspirierten Luft ($FiO_2$) rapide erhöht wurde, als Tracersubstanz der zerebralen Durchblutung. Die Anflutung des "Tracers" in der zerebralen Zirkulation wird mittels NIRS detektiert und, auf der Grundlage des Fick'schen Prinzips, durch Vergleich mit der peripher mittels Pulsoxymetrie ermittelten Anflutung der zerebrale Blutfluß errechnet (Edwards, A. D. et al.: J. Appl. Physiol. 75 (1993), 1884-1889; Elwell, C. E. et al: Adv. Exp. Med. Biol. 317 (1992), 235-245; Elwell, C. E. et al.: J. Apll. Physiol. 77 (1994), 2753-2760; Elwell, C. E. et al.: Acta Neurochir. 59 (1993), 74-80). Die Methodik wird durch Vergleich mit einer plethysmographischen Blutflußmessung am Unterarm validiert (Edwards, A. D. et al.: J. Appl. Physiol. 75 (1993), 1884-1889).

Nachteilig bei der von Edwards beschriebenen Methode ist, daß die Anflutung der Tracersubstanz sich nicht exakt steuern läßt, da die rasche Oxygenierung des Hämoglobins bei Erhöhung der $FiO_2$ von der Ventilation abhängt. Außerdem wird die Tracersubstanz selbt bei raschestem Anstieg der $HbO_2$ nie den Charakter einer schlagartigen Erhöhung der Tracerkonzentration erreichen, die für eine optimale Bestimmung des CBF nötig ist. Für das Erreichen eines meßbaren Anstiegs der $HbO_2$ durch Erhöhung der $FiO_2$ ist vorab eine Erniedrigung der $HbO_2$ durch Hypoventilation nötig. Diese ist jedoch den zumeist traumatisierten Patienten, bei denen eine Messung des CBF klinisch relevant wäre, in der Regel nicht zuzumuten.

Aus diesen Gründen modifizierte Roberts et al. (Roberts, I. et al.: The Lancet 342 (1993), 1425) die Methodik, indem sie anstatt des $HbO_2$ einen intravenös applizierten Farbstoff als Tracersubstanz einsetzten. Der Farbstoff Indocyaningrün (1-[Sulfobutyl]-3,3-dimethyl-2-(7-[-[4-sulfobutyl]-3,3-dimethyl-4,5-benzo-indolinyliden-[2]]-heptatrien-[1,3,5]-yl]4,5,benzoindolium) mit einem Molekulargewicht von 774,97 Dalton (Cherrick, G. R. et al.: J. Clin. Invest. 39 (1960), 592-600) verfügt über ein Absorptionsmaximum, das bei 805 mm und somit im Spektrum des nah-infraroten Lichtes liegt (Landsman, M. L. J. et al.: J. Appl. Physiol. 40 (1976), 575-583). Nach intravenöser Applikation ist die Verteilung von Indocyaningrün durch 95%ige Bindung an Plasmaproteine (Muckle, T. J.: Biochem. Med. 15 (1976), 17-21), insbesondere $\alpha$-Lipoproteine, streng auf das intravaskuläre Kompartiment beschränkt (Cherrick, G. R. et al.: J. Clin. Invest. 39 (1960), 592-600). Dadurch eignet es sich hervorragend als intravasaler Tracer. Da es darüberhinaus rasch über die Leber aus dem Kreislauf eliminiert wird (Cherrick, G. R. et al.: J. Clin. Invest. 39 (1960), 592-600), gestatten sich wiederholte Messungen in kurzen Zeitabständen. Die besonderen lichtabsorbierenden Eigenschaften von Indocyaningrün im nah-infraroten Spektrum ermöglichen seine Detektion mittels Nah-Infrarot-Spektroskopie.

Erstmals setzten Proctor et al. 1984 (Proctor, H. J. et al.: Surgery 96 (1984), 273-279) die intravenöse Applikation von Indocyaningrün als Tracersubstanz zur Bestimmung der zerebralen Durchblutung mittels Nah-Infrarot-Spektroskopie ein. Sie errechneten das Integral unter der mittels NIRS gemessenen Anflutungskurve nach intravenöser Applikation eines definierten Bolus von Indocyaningrün.

Roberts et al. (Roberts, I. et al.: The Lancet 342 (1993), 1425) versuchten, anstelle des körpereigenen Tracers $HbO_2$ eine intravenöse Bolusinjektion von Indocyaningrün als Tracersubstanz zu verwenden und wiederum anhand des Fick'-schen Prinzips den zerebralen Blutfluß zu berechnen. Hierzu wurde die Anflutung von Indoyaningrün im zerebralen Gefäßsystem mittels NIRS detektiert und simultan die arterielle Anflutung des Farbstoffes über einen invasiv implantierten arteriellen Katheter registriert. Der zerebrale Blutfluß wurde sodann als CBF = $k(Q(t)/(_0\int^t(Pa)dt))$ bestimmt, wobei CBF den zerebralen Blutfluß [ml/(100 g $\times$ min)], k die Konstante bestehend aus dem Molekulargewicht von Indocyaningrün und der Dichte des zerebralen Gewebes, Q die mittels NIRS gemessene Akkumulation des Tracers im Gehirn und P(a) die invasiv über einen intravaskulären, fiberoptischen Katheter gemessene, arterielle Konzentration des Tracers sind.

Beide Verfahren zur Messung des zerebralen Blutflusses mittels Nah-Infrarot-Spektroskopie und intravenöser Applikation eines Tracerfarbstoffes wurden nicht validiert, d. h. die erhaltenen Meßwerte wurden nicht mit einer davon unabhängigen Methode verglichen. In einer kürzlich durchgeführten Studie (Kuebler, W.M. et al.: Int. J. Microcirc.: Clin. Exp. 16 S1 (1996), 223) konnte erstmals nachgewiesen werden, daß die mit einer der beiden beschriebenen Methoden erhaltenen Meßwerte in keiner Weise die zerebrale Durchblutung widerspiegeln, sondern vielmehr zumeist Ausdruck des Herzzeitvolumens sind, von dem die Gehirndurchblutung aufgrund ihrer Fähigkeit zur Autoregulation über einen weiten Bereich unabhängig ist.

Nachteilig an der von Edwards et al. beschriebenen Methode ist außerdem, daß es sich hierbei um keine nicht invasive Methode handelt, da sie der Implantation eines zusätzlichen intravaskulären fiberoptischen Meßkatheters bedarf und somit nicht zur raschen, nicht invasiven Messung des zerebralen Blutflusses am Krankenbett geeignet ist.

Ein neues Meßverfahren des zerebralen Blutflusses mit Hilfe einer Doppelindikatorverdünnungsmethode (Mielck, F. et al.: Abstract Book, The European Association of Cardiothoracic Anaesthesiologists 11 (1996), -7) ermöglicht die rasche und wiederholte Bestimmung des CBF am Krankenbett, beruht jedoch auf einer invasiven Messung mittels intravaskulärer Verweilkatheter und bietet keine regionale Auflösung.

Bislang ist demnach keine Methode bekannt, die die rasche, wiederholte, nicht invasive und regional auflösende Messung des zerebralen Blutflusses (CBF), dessen adäquate Regulation unabdingbare Voraussetzung einer intakten neuronalen Aktivität des Gehirns ist, am Patientenbett gestattet.

Aufgabe der vorliegenden Erfindung ist, eine Anordnung zur Messung des zerebralen Blutflusses mittels Nah-Infrarot-Spektroskopie unter intravenöser Applikation eines Tracerfarbstoffes zur Verfügung zu stellen, die die aus dem Stand der Technik bekannten Nachteile nicht aufweist und nicht invasive, rasche, anwenderfreundliche, kostengünstige und wiederholte Messungen des zerebralen Blutflusses am Krankenbett ohne Beeinträchtigung der Patientenversorgung (z. B. durch Hypoventilation) simultan für zwei oder mehr Hirnregionen, so z.B. für beide Gehirnhemisphären, gestatten.

Diese Aufgabe wird durch die Anordnung gemäß Anspruch 1 gelöst. Weitere Ausgestaltungen sind den abhängigen Ansprüchen zu entnehmen.

Erfindungsgemäß wird die Anflutung eines intravenös injizierten Bolus einer Tracersubstanz mit Absorptionseigenschaften im nah-infraroten Spektrum simultan und nicht invasiv in mehreren Hirnregionen, etwa in beiden Hirnhemisphären mittels Nah-Infrarot-Spektroskopie und im arteriellen Blut des systemischen Kreislaufs mittels pulsdensitometrischer arterieller Farbstoffmessung erfaßt. Durch Entfaltung der arteriellen und der zerebralen Anflutungskinetiken wird die transzerebrale Transportfunktion errechnet, anhand deren Kinetik ein Blutflußindex bestimmt wird, der dem zerebralen Blutfluß direkt proportional ist.

Das der Erfindung zugrunde liegende Prinzip der Durchblutungsmessung ist das folgende: Die Passage eines Tracerbolus durch den zerebralen Kreislauf ändert die optische Dichte des Gehirns im Absorptionsbereich der eingesetzten Tracersubstanz. Diese Änderung der optischen Dichte über die Zeit kann mittels Nah-Infrarot-Spektroskopie quantifiziert werden und entspricht der zerebralen Farbstoffkurve. Die transzerebrale Transportfunktion, die die charakteristischen Übertragungseigenschaften des Systems beschreibt und in der Terminologie der Ingenieurwissenschaften als Gewichtsfunktion bezeichnet wird, kann durch "Entfaltung" der zerebralen Farbstoffkurve mit der nicht invasiv pulsdensitometrisch gemessenen arteriellen Farbstoffkurve im systemischen Kreislauf bestimmt werden. Den Zusammenhang zwischen Transportfunktion g(t), arterieller a(t) und zerebraler c(t) Farbstoffkurve drückt das folgende Faltungsintegral aus:

$$c(t) = {}_0\!\int^\infty g(t-u)\ a(u)\ du,$$

wobei u eine Hilfsvariable der Integration ist.

Die Ermittlung der transzerebralen Transportfunktion g(t) durch "Entfaltung" von a(t) und c(t) kann durch modellfreie Verfahren durchgeführt werden; numerisch günstiger ist jedoch die Verwendung von Modellfunktionen. Ist die grundsätzliche Form der Transportfunktion bekannt und durch eine Funktion beschreibbar, so können für gemessene Kurvenpaare von a(t) und c(t) die Parameter der zugehörigen Transportfunktionen durch ein iteratives nicht lineares Anpassungsverfahren ermittelt werden. Hierzu werden nach dem Prinzip der kleinsten Quadrate unter iterativer Variation der Parameter der Transportfunktion g(t) die Quadrate der Differenzen zwischen gemessenen zerebralen Konzentrationen und dem Faltungsergebnis der arteriellen Kurve mit der Transportfunktion minimiert, die durch folgenden Ausdruck bestimmt werden:

$$[c(t) - {}_0\!\int^\infty g(t-u)\ a(u)\ du]^2$$

Dabei ist zu beachten, daß sich aufgrund der unterschiedlichen Meßverfahren der zerebralen und der arteriellen Farbstoffkurve deren Dimensionen unterscheiden und auch nicht wechselseitig ineinander überführen lassen. Entsprechend ergibt sich für die transzerebrale Transportfunktion g(t) die Dimension [ml/min].

Zur Beschreibung intravasaler Farbstofftransportfunktionen haben sich logarithmische Normalverteilungen bewährt. Aus diesem Grund kann für g(t) folgender Ansatz gewählt werden:

$$g(t)=g_{lognor}(t,mtt,\sigma)=(1/\sqrt{2\pi}\sigma t)e^{-(lnt/mtt+\sigma^2/2)^2/2\sigma^2}$$

wobei mtt die mittlere Transitzeit des Farbstoffes und σ die relative Dispersion des Farbstoffes ist.

Dabei beziehen sich mittlere Transitzeit des Farbstoffes wie auch die Transportfunktion selbst nicht auf den arterio-venösen Transit des Indikators, sondern lediglich auf dessen Anflutung im Gewebe, in dem mittels Nah-Infrarot-Spektroskopie die Farbstoffkurve ermittelt wird.

Bestehen wie beim Gehirn mit grauer und weißer Substanz unterschiedlich schnell durchblutete intravaskuläre Kompartimente, kann die Transportfunktion durch die Summe zweier logarithmischer Normalverteilungen gemäß folgender Formel repräsentiert werden.

$$g(t) = \Sigma\ \alpha;\ g_{log\,nori}\ (t,\ mtt_1,\ \sigma_i)$$

Aus der somit bestimmten transzerebralen Transportfunktion des Tracers g(t) wird nun folgendermaßen auf die zerebrale Durchblutung rückgeschlossen.

Aus dem Quotienten von Anflutungsmaximum und Anflutungszeit des Farbstoffes in der transzerebralen Transportfunktion nach Bolusinjektion des Farbstoffes wird ein Blutflußindex errechnet, der den tatsächlichen Durchblutungsverhältnissen des Organs direkt proportional ist:

$$\text{Blutflußindex [ml/100 g} \times \text{s}^{-1}] =$$

$$\text{Anflutungsmaximum/Anflutungszeit mit}$$

$$\text{Anflutungszeit [S]} = t_{(y\,\%\,des\,Signalanstiegs)} - t_{(x\,\%\,des\,Signalanstiegs)},$$

wobei t die Zeit [s] und x und y Variablen sind, die Anfang und Ende des untersuchten zeitlichen Anflutungsintervalls der transzerebralen Transportfunktion definieren.

Die Erfindung wird im folgenden mit Bezug auf die Figuren näher erläutert.

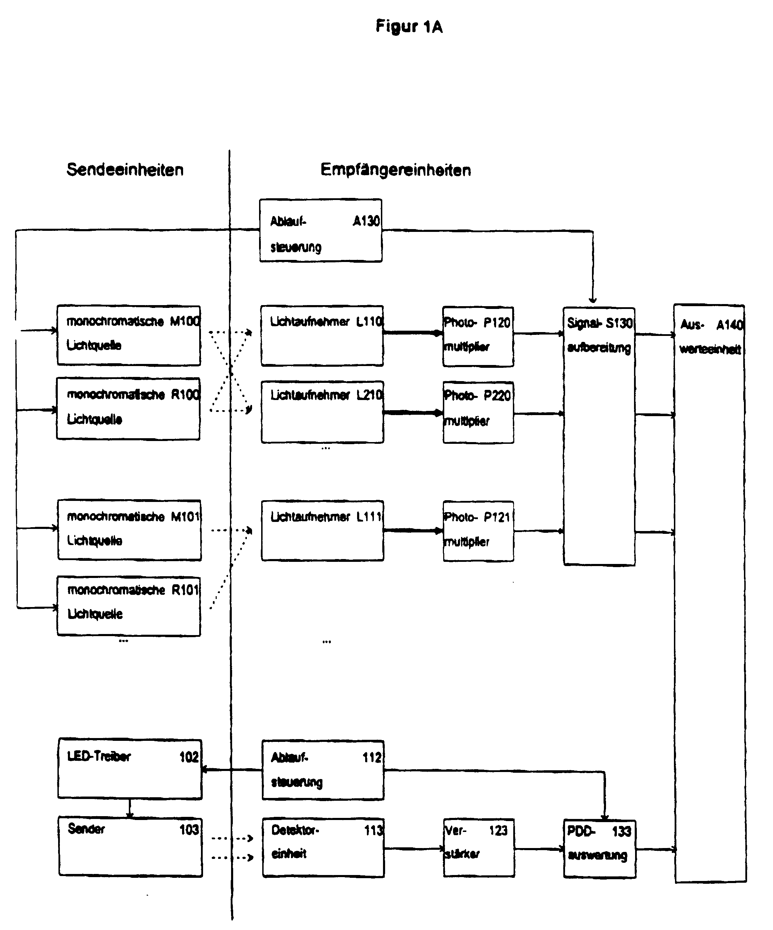

Figur 1A zeigt das Blockschaltbild der erfindungsgemäßen Vorrichtung. Diese besteht aus einem Mehrkanalgerät mit zwei oder mehr Spektroskopen, jeweils bestehend aus einer gepulsten monochromatischen Lichtquelle (M100, M101) mit einer Wellenlänge im Bereich des Absorbtionsmaximums der Tracersubstanz und einer weiteren gepulsten Lichtquelle (R100, R101) die monochromatisches Licht in einem Wellenlängenbereich emittiert, der von der Tracersubstanz möglichst gering absorbiert wird. Das gestreute und reflektierte Licht wird von einem oder mehreren Lichtaufnehmern (L110, L210 und L111) aufgenommen und über Lichtwellenleiter an Photomultiplier (P120, P220 und P121) weitergegeben, wo das optische Meß- und Referenzsignal in ein elektrisches gewandelt wird. Die Koordination zwischen den gepulsten Lichtquellen und der Signalaufbereitung (S130) übernimmt dabei die Ablaufsteuerung (A130). Die in S130 bestimmten zerebralen Farbstoffkurven werden an die Auswerteeinheit (A140), z.B. digital über eine RS232-Schnittstelle, weitergeleitet. Der Auswerteeinheit stehen zudem die Informationen über die arteriell gemessene Farbstoffkurve zur Verfügung, die nicht invasiv pulsdensitometrisch gemessen wird. Ein Ausführungsbeispiel der Anordnung zur Pulsdensitometrie, die als bekannt vorausgesetzt werden kann, ist im unteren Teil der Figur 1A gezeigt.

Figur 1B zeigt schematisch das Ausführungsbeispiel der erfindungsgemäßen Anordnung zur nicht-invasiven Messung der zerebralen Durchblutung in beiden Hirnhemisphären mittels Nah-Infrarot-Spektroskopie, Pulsdensitometrie und intravenöser Applikation eines Tracerfarbstoffes.

Die Figuren 2A bis 2C zeigen beispielhaft mittels Pulsdensitometrie gemessene arterielle und mittels Nah-Infrarot-Spektroskopie gemessene zerebrale Farbstoffkurven.

Figur 3 zeigt beispielhaft eine durch Entfaltung der arteriellen und zerebralen Farbstoffkurven ermittelte transzerebrale Transportfunktion.

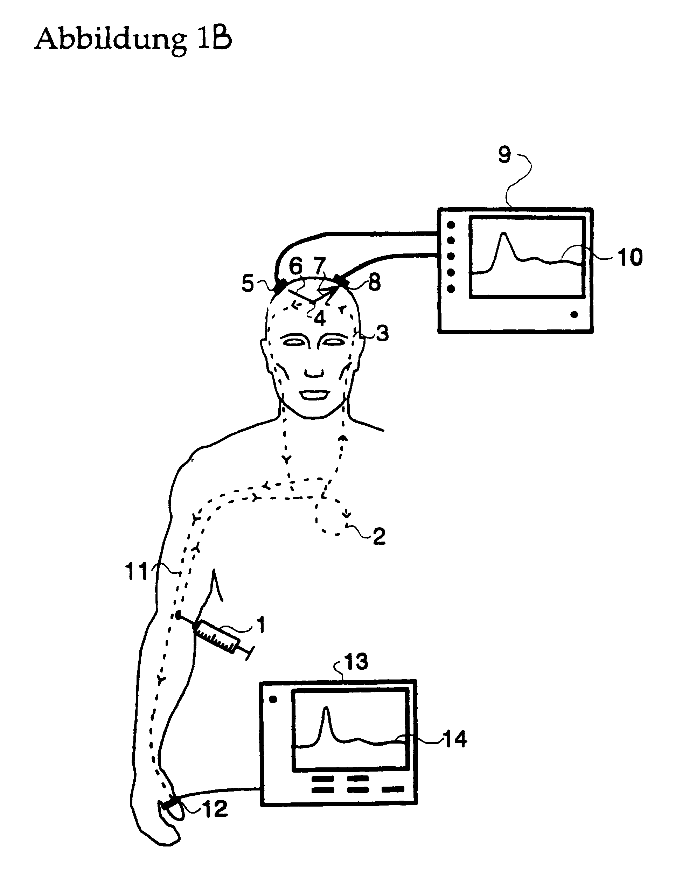

Gemäß Figur 1B gelangt ein Bolus eines intravenös injizierten Tracerfarbstoffes mit Absorptionseigenschaften im nah-infraroten Spektrum (z. B. Indocyaningrün) (1) über das venöse Gefäßsystem, das rechte Herz und die Lungenstrombahn zum linken Herzen, das ihn in die arterielle Blutbahn auswirft. Ein Teil des Farbstoffbolus gelangt so über die Halsschlagadern (Aa. carotides) (2) in das Gehirn und passiert dessen Gefäßsystem.

Über jeder Hirnhemisphäre emittieren zwei oberflächlich auf der Kopfschwarte plazierte monochromatische Lichtquellen (3) im Wellenlängenbereich des nah-infraroten Spektrums - wovon eine (Meßwellenlänge) dem Absorptionsmaximum der eingesetzten Tracersubstanz möglichst entspricht, während die andere (Referenzwellenlänge) eine Wellenlänge aufweist, bei der die eingesetzte Tracersubstanz möglicht geringe Absorptionseigenschaften aufweist - nah-infrarotes Licht (4), das das darunterliegende Hirngewebe nicht-invasiv durchdringt. Im Gewebe wird das emittierte nah-infrarote Licht beider Wellenlängen (Meß- und Referenzwellenlänge) zum Teil von körpereigenen Chromophoren (Hämoglobin, Cytochrome, usw.) absorbiert und teilweise als Streulicht reflektiert. Letzteres wird auf jeder Hemisphäre durch mindestens einen ipsilateral oberflächlich auf der Kopfschwarte plazierten Lichtaufnehmer (5), der über Glasfaserkabel an einen Photomultiplier gekoppelt ist, erfaßt und quantifiziert.

Die Passage der intravenös applizierten Tracersubstanz durch das zerebrale Gefäßsystem ändert die optische Dichte des Gewebes im Absorptionsbereich des Farbstoffes, d.h. im Meßbereich der Meß-, jedoch nicht der Referenzwellenlänge. Aus dem Quotienten der Signale an den Photmomultipliern für Meß- und Referenzwellenlänge läßt sich für jede Hirnhemisphäre die zerebrale Farbstoffkurve c(t) (6,7) über die Zeit bestimmen.

Simultan gelangt ein Teil des vom linken Herzen ausgeworfenen Farbstoffbolus in das periphere arterielle Gefäßsystem (8), wo mittels nicht-invasiver pulsdensitometrischer Messung (9) die arterielle Farbstoffkurve bestimmt wird (10).

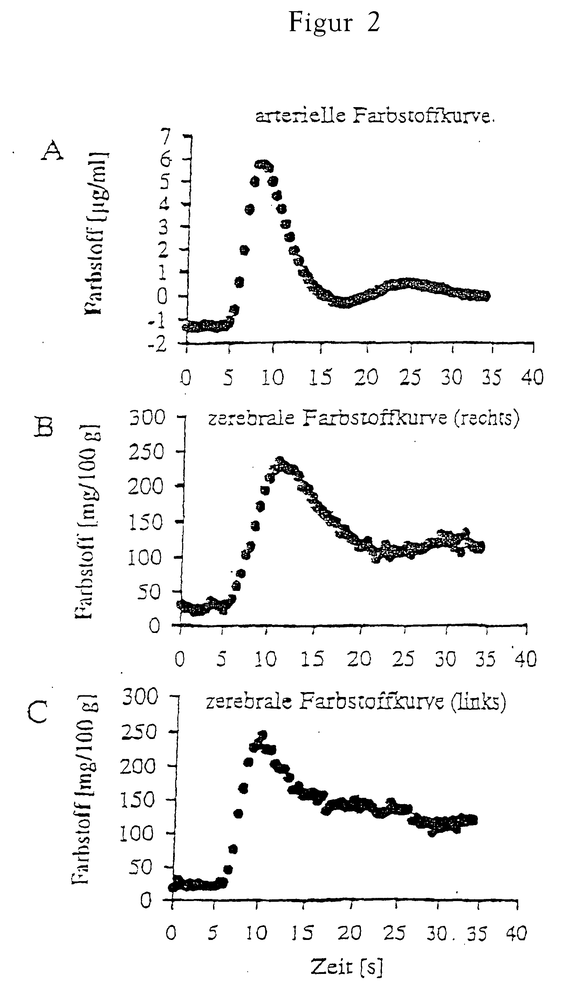

Nach venöser Bolusinjektion einer ausreichenden Indikatormenge werden die in Figur 2 beispielhaft dargestellten Farbstoffkurven registriert und über ein Analogsignal auf dem Rechner aufgezeichnet. Mittels pulsdensitometrischer Messung wird die arterielle Anflutung des Farbstoffes nicht invasiv bestimmt. Die zerebralen Farbstoffkurven werden für die rechte und linke Hirnhemisphäre getrennt mittels Nah-Infrarot-Spektroskopie detektiert. Dabei ergeben sich typischerweise die in Figur 2 dargestellten Indikatorverdünnungskurven; und zwar die arterielle Farbstoffkurve gemäß Figur 2A und die zerebralen Farbstoffkurven für die rechte Gehirnhemisphäre gemäß Figur 2B und für die linke Gehirnhemisphäre gemäß Figur 2C.

Nach Korrektur für das Hintergrundsignal wird die arterielle Farbstoffkurve mit beiden zerebralen Farbstoffkurven zeitlich so abgeglichen, daß sich die Anflutungsbeginne der jeweiligen Kurven überlagern. Durch Entfaltung einer jeden zerebralen Farbstoffkurve mit dem entsprechenden arteriellen Signal kann die jeweilige transzerebrale Transportfunktion für jede Hirnhemisphäre bestimmt werden.

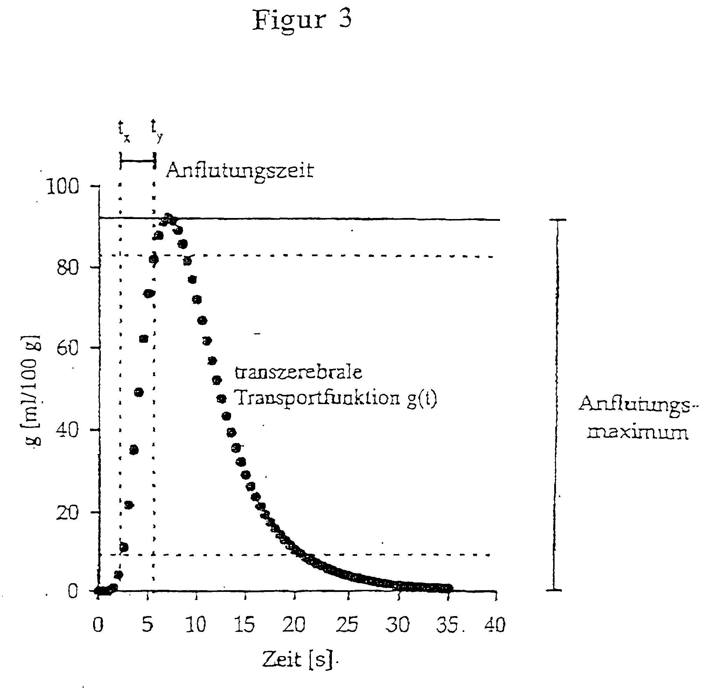

Figur 3 zeigt beispielhaft eine transzerebrale Transportfunktion g(t), welche die charakteristischen Übertragungseigenschaften des zerebralen Gefäßsystems widergibt.

Die intravasale Anflutung des Farbstoffes führt in der transzerebralen Transportfunktion zu einem raschen Anstieg des Meßsignals, das innerhalb weniger Sekunden sein Maximum (Anflutungsmaximum) erreicht (in Figur 3 bei 92,28 ml/100g).

Die Anflutungszeit entspricht dem Zeitintervall $t_z$, in das die Werte z mit

x % des Signalanstiegs < z < y % des Signalanstiegs fallen.

In den Meßbeispielen gemäß Figur 2 und 3 wurden x und y definiert als:

x = 10 % des Signalanstiegs = 9,23 ml/100 g und
y = 90 % des Signalanstiegs = 83,05 ml/100 g.

Entsprechend gilt für das Beispiel gemäß Figur 3
$t_x$ = 2,40 s und $t_y$ = 5,58 s. Entsprechend beträgt die Anflutungszeit
$t_z$ = $t_y$ - $t_x$ = 3,18 s.
In dem Ausführungsbeispiel ergibt sich somit ein Blutflußindex von

$$\text{Blutflußindex [ml/100 g x s}^{-1}] = \text{Anflutungsmaximum}/(t_y - t_x)$$

$$= 92,28 \text{ ml/100 g}/3,18 \text{ s} = 29,02 \text{ ml/100 g x s}^{-1}.$$

Die vorliegende Erfindung stellt eine Anordnung zur Verfügung, welche benutzerfreundliche, nicht invasive, schnelle und wiederholte Messungen des zerebralen Blutflusses am Krankenbett sowie die sofortige (on-line) und leichtverständliche Erhebung und Interpretation der Meßergebnisse ermöglicht. Die erfindungsgemäße Anordnung zeichnet sich insbesondere' durch geringe Gerätekosten, hohe Mobilität und entsprechend rasche Verfügbarkeit aus.

Im Gegensatz zu dem vorbekannten Verfahren bzw. der Vorrichtung der nicht-invasiven Bestimmung des zerebralen Blutflusses mittels Nah-Infrarot-Spektroskopie und intravenöser Applikation eines Tracerfarbstoffes nach Roberts et al. (Roberts, 1. et al.: The Lancet 342 (1993), 1425) besteht die erfindungsgemäße Vorrichtung aus 2 Spektroskopen und erlaubt so die simultane Messung der relativen Hirndurchblutung über beiden Hirnhemisphären. Das von Roberts et al. verwendete Nah-infrarot-Spektroskop NIRO 500 der Fa. Hamamatsu Photonics umfast 4 gepulste Laser im nah-infraroten Spektralbereich, wohingegen in der von uns beschriebenen Vorrichtung über jeder Hirnhemisphäre lediglich 2 gepulste monochromatische Lichtquellen, jeweils für eine Meß- und eine Referenzwellenlänge im nah-infraroten Spektralbereich, verwendet werden.

Schließlich wird die arterielle Farbstoffkurve in der von Roberts et al. beschriebenen Vorrichtung mittels eines invasiv intraarteriell implantierten fiberoptischen Katheters gemessen. Die von uns beschriebene Vorrichtung beruht hingegen auf der nicht-invasiven Messung der arteriellen Farbstoffkurve mittels eines pulsdensitometrischen Meßgerätes. Die Invasivität der von Roberts et al. beschriebenen Vorrichtung bzw. des Verfahrens stellt einen wesentlichen Unterschied gegenüber der von uns beschriebenen Vorrichtung bzw. dem Verfahren dar, da die Implantation eines fiberoptischen Katheters ein chirurgisches Verfahren mit den medizinischen Risiken von Infektion, Perforation oder Thrombose/Embolie darstellt; wohingegen die von uns beschriebene pulsdensitometrische Messung nicht-invasiv erfolgt und somit keine der genannten Risiken in sich trägt.

Gegenüber der ebenfalls auf einer Dekonvolution beruhenden Messung des zerebralen Blutflusses mit Hilfe einer Doppelindikatorverdünnungsmethode nach Hoeft (internationales AZ PCT/DE95/01690) bzw. Mielck et al. (Mielck, F. et al.: Abstract Book, The European Associaton of Cardiothoracic Anaesthesiologists 11 (1996), 7) unterscheidet sich die Anordnung gemäß der Erfindung insofern, als das Verfahren nach Hoeft ein Doppelindikatorverfahren mittels Bestimmung einer Farbstoffkinetik sowie einer Thermodilution darstellt, wobei der zerebrale Blutflug im besonderen allein auf der Thermodilutionsmethode (hochdiffusibler Tracer) beruht. Hingegen stützt sich die Messung mit der Anordnung nach der Erfindung einzig auf die Bestimmung einer Farbstoffkinetik (intravasaler Tracer). Vorrichtung sowie Meßverfahren der Hoeft'schen Anmeldung unterscheiden sich komplett von der von uns beschriebenen, insbesondere hinsichtlich der Invasivität der Messung, die nach dem Hoeft' schen Verfahren die chirurgische Implantation zweier kombinierter Fiberoptik-Thermistor-Katheter einschließt. Die gemeine Bestimmung einer transzerebralen Transportfunktion des jeweilig verwendeten Indikators durch Berechnung eines sog. Faltungsintegrals erfolgt in beiden Fällen mittels eines standardisierten Dekonvolutionsverfahrens. Die dadurch errechneten Transportfunktionen die die charakteristischen Übertragungseigenschaften eines Systems beinhalten, sind als sog. Gewichtsfunktionen in Natur- und Ingenieurwissenschaften weit verbreitet (Hoeft, A. Dilutionstechniken und Ficksches Prinzip. In: Monitoring in Anästhesie und Intensivmedizin, edited by W.F.List, H. Metzler, und T. Pasch. Berlin, Heidelberg, New York: Springer-Verlag, 1995, p. 250-291).

Im Gegensatz zu Methoden der Bestimmung der globalen Hirndurchblutung erfaßt die Anordnung gemäß der Erfindung die Hirndurchblutung gesondert für rechte und linke Hirnhemisphäre, d.h. die Erfindung ermöglicht eine regionale Auflösung, die die quantitative Bestimmung der relativen Durchblutung der einzelnen Gehirnhemisphären erlaubt. Dadurch bietet die Anordnung gemäß der Erfindung die Möglichkeit, regionale Verteilungen bzw. Verteilungsstörungen der Hirndurchblutung zwischen rechter und linker Hirnhemisphäre zu erfassen.

Die Passage unterschiedlicher Tracersubstanzen mit Absorptionseigenschaften im nah-infraroten Spektrum kann mittels Nah-Infrarot-Spektroskopie erfaßt werden. Entsprechend kann die Passage eines Tracers mit Absorptionseigenschaften im nah-infraroten Spektrum bei verschiedenen Wellenlängen im nah-infraroten Bereich erfaßt werden. Entsprechend kann die Passage eines Tracers mit Absorptionseigenschaften im nah-infraroten Spektrum bei Einsatz verschiedener Mengen und Konzentrationen der Tracersubstanz mittels NIRS erfaßt werden.

Entsprechend kann die Passage eines Tracers mit Absorptionseigenschaften im nah-infraroten Spektrum mittels mehrerer räumlich angeordneter Lichtaufnehmer erfaßt werden, wodurch eine regionale bzw. Tiefenauflösung der Blutflußmessung erreicht werden kann.

Die Bestimmung der Organdurchblutung mittels des erfindungsgemäßen Anordnung ist prinzipiell nicht auf die Bestimmung des zerebralen Blutflusses limitiert. Vielmehr kann mit Hilfe der erfindungsgemäßen Vorrichtung die Passage einer intravasalen Tracersubstanz mit Absorptionseigenschaften im nah-infraroten Spektrum in verschiedenen Organen zur nicht invasiven Bestimmung der Durchblutung erfaßt werden.

In Ergänzung des in der vorliegenden Erfindung eingesetzten Algorithmus und unter Miteinbeziehung der mittels der erfindungsmäßigen Anordnung nicht-invasiv gemessenen arteriellen und zerebralen Farbstoffkurven lassen sich zudem weitere zirkulatorische Parameter, d.h. das Herzzeitvolumen nach der Stewart-Hamilton-Methode, das intrathorakale Blutvolumen als Produkt der mittleren Transitzeit (mtt) und des Herzzeitvolumens sowie - ebenfalls mit regionaler Auflösung - das relative zerebrale Blutvolumen als Produkt der mittleren Transitzeit (mtt) der Transportfunktion g(t) und dem relativen Blutflußindex der entsprechenden Region, quantifizieren.

**Patentansprüche**

1. Anordnung zur nicht invasiven Bestimmung des Blutflusses durch ein Organ nach intravenöser Applikation eines Indikatorfarbstoffes mit Absorptionseigenschaften im nah-infraroten Spektrum, **gekennzeichnet durch** ein nicht invasives pulsdensitometrisches Meßgerät zur gesonderten Messung der arteriellen Farbstoffkurve a(t) im systemischen Kreislauf und zwei oder mehr auf das Organ auszurichtende Nah-Infrarot-Spektroskope zur Ermittlung einer Organ-Farbstoffkurve c(t), jeweils bestehend aus zwei oder mehr gepulsten monochromatischen Lichtquellen im Wellenlängenbereich des nah-infraroten Spektrums - wovon eine Meßwellenlänge dem Absorptionsmaximum des eingesetzten Indikatorfarbstoffs möglichst entspricht, während eine Referenzwellenlänge eine Wellenlänge aufweist, bei der der eingesetzte Indikatorfarbstoff möglichst geringe Absorptionseigenschaften aufweist, sowie jeweils einem oder mehreren zugehörigen Lichtaufnehmern als Meßsensoren, die über ein Glasfaserkabel mit einem Photomultiplier verbunden sind, zur gesonderten Messung der Organ-Farbstoffkurven mit regionaler Auflösung.

2. Anordnung nach Anspruch 1, **dadurch gekennzeichnet, daß** das pulsdensitometrische Meßgerät und die Nah-Infrarot-Spektroskope zur Ermittlung der Transportfunktion g(t) anhand des Faltungsintegrals

$$c(t) = {}_0\!\int^\infty g(t-u)\ a(u)\ du,$$

wobei u eine Hilfsvariable der Integration ist, durch Entfaltung der gemessenen arteriellen Farbstoffkurven a(t) und der durch die Nah-Infrarot-Spektroskope ermittelten Organ-Farbstoffkurve c(t) mit einem Rechner verbunden sind.

3. Anordnung nach Anspruch 2, in der der Rechner die Transportfunktionen g(t) aus der gemessenen arteriellen Farbstoffkurve a(t) und Organ-Farbstoffkurve c(t) mit Hilfe eines iterativen, nichtlinearen Anpassungsverfahrens ermittelt, wobei der grundsätzliche Verlauf der Transportfunktion durch parametrisierte Modellfunktionen beschrieben wird.

4. Anordnung nach Anspruch 3, **dadurch gekennzeichnet, daß** nach dem Verfahren der kleinsten Quadrate die Parameter dergestalt variiert werden, bis der Fehler, der durch die Quadrate der Differenzen

$$[c(t) - {}_0\!\int^\infty g(t-u)\ a(u)\ du]^2$$

bestimmt ist und den Unterschied zwischen der nah-infrarot spektroskopisch gemessenen Organ-Farbstoffkurve c(t) und dem Faltungsergebnis der pulsdensitometrisch gemessenen arteriellen Farbstoffkurve a(t) wiedergibt, minimiert ist.

5. Anordnung nach Anspruch 4, in der als Modellfunktion für die intravasale Farbstofftransportfunktion g(t) eine logarithmische Normalverteilung der Form

$$g(t) = g_{lognor}(t, mtt, \sigma) = (1/\sqrt{2\pi}\sigma t)e^{-(\ln t/mtt + \sigma^2/2)^2/2\sigma^2}$$

gewählt wird, wobei die zu variierenden Parameter durch die mittlere Transitzeit der Farbstofflösung mtt und die relative Dispersion $\sigma$ gebildet werden.

6. Anordnung nach Anspruch 4 und/oder 5, in der die Modellfunktion für N unterschiedlich stark durchblutete Kompartimente durch die Summe von N logarithmischer Normalverteilungen der Form

$$g(t) = \Sigma\ \alpha_i\ g_{log\ nori}\ (t, mtt_i, \sigma_i)$$

mit $\alpha_i > 0$ und der Nebenbedingung $\Sigma\ \alpha_i = 1$ modifiziert wird.

**7.** Anordnung nach mindestens einem der Ansprüche 1 bis 6, in der anhand der Organ-Farbstofftransportfunktion g(t) ein Blutflußindex gemäß der Beziehung

$$\text{Blutflußindex} = \text{Anflutungsmaximum/Anflutungszeit}$$

bestimmt wird, der der tatsächlichen Organ-Durchblutung direkt proportional ist, wobei für die Ermittlung der Anflutungszeit

$$\text{Anflutungszeit [s]} = t_{(y\,\%\,\text{des Signalanstiegs})} - t_{(x\,\%\,\text{des Signalanstiegs})},$$

gilt, wobei t die Zeit [s] und x und y Variablen sind, die Anfang und Ende des untersuchten zeitlichen Anflutungsintervalls der Transportfunktion definieren.

**8.** Anordnung nach mindestens einem der Ansprüche 1 bis 7, **gekennzeichnet durch** mehrere mit dem Rechner verbundene Nah-Infrarot-Spektroskope zur Erfassung zerebraler Farbstoffkurven simultan über verschiedenen Hirnarealen zur regionalen Messung des Blutflusses in verschiedenen Hirnarealen.

**9.** Anordnung nach mindestens einem der Ansprüche 1 bis 8, **dadurch gekennzeichnet, daß** durch nah-infrarot spektroskopische Messung der Passage eines Indikatorfarbstoffs mit Absorptionseigenschaften im nah-infraroten Spektrum mittels mehrerer räumlich angeordneter Lichtaufnehmer eine regionale bzw. Tiefenauflösung der Blutflußmessung ermöglicht wird.

**10.** Anordnung nach mindestens einem der Ansprüche 1 bis 9, in der verschiedene Farbstoffe in unterschiedlichen Mengen und Konzentrationen als Indikatorfarbstoffe zum Einsatz kommen und nah-infrarot-spektroskopische Messungen bei verschiedenen Wellenlängen durchgeführt werden.

**11.** Anordnung nach mindestens einem der Ansprüche 1 bis 10, **dadurch gekennzeichnet, daß** weitere zirkulatorische Parameter, i.e. das Herzzeitvolumen nach der Stewart-Hamilton-Gleichung, das intrathorakale Blutvolumen als Produkt der mittleren Transitzeit (mtt) und des Herzzeitvolumens sowie - ebenfalls mit regionaler Auflösung - das relative Blutvolumen als Produkt der mittleren Transitzeit (mtt) der Transportfunktion g(t) und dem relativen Blutflußindex der entsprechenden Region quantifiziert werden.

## Claims

**1.** A device for non-invasively determining blood flow through an organ after intravenous application of an indicator dye with absorption properties in the near-infrared spectrum, **characterised by** a non-invasive pulse-densitometry instrument for separate measurement of the arterial dye curve a(t) in the systemic circulation and two or more near-infrared spectroscopes to be directed onto the organ to establish an organ dye curve c(t), each comprising two or more pulsed monochromatic light sources in the wavelength range of the near-infrared spectrum - of which one measuring wavelength corresponds as far as possible to the absorption maximum of the indicator dye used, while a reference wavelength has a wavelength at which the indicator dye used has the least possible absorption properties, and in each case one or more associated light detectors as measuring sensors which are connected by a fibreglass cable to a photo-multiplier for separate measurement of the organ dye curves with local resolution.

**2.** The device of claim 1, **characterised in that** the pulse-densitometry instrument and the near-infrared spectroscopes for establishing the transporting function g(t) based on the folding integral

$$c(t) = {}_0\!\int^{\infty} g(t-u)\ a(u)\ du,$$

where u is an additional variable in the integration, by unfolding the measured arterial dye curves a(t) and the organ dye curve c(t) established by the near-infrared spectroscopes are connected to a computer.

3. The device of claim 2, wherein the computer establishes the transporting functions g(t) from the measured arterial dye curve a(t) and the organ dye curve c(t) by means of an iterative, non-linear adaptation process, the basic course of the transporting function being described by parametrised modelling functions.

4. The device of claim 3, **characterised in that** the parameters are varied by the method of least squares until the error defined by the squares of the differences

$$[c(t) - {}_0\!\int^{\infty} g(t-u)\ a(u)\ du]^2$$

and reproducing the difference between the organ dye curve c(t) measured by near-infrared spectroscopy and the result of folding the arterial dye curve a(t) measured by pulse densitometry is minimised.

5. The device of claim 4, wherein a logarithmic normal distribution of the form

$$g(t) = g_{lognor}(t,mtt,\sigma) = (1/\sqrt{2\pi}\sigma t)e^{-(lnt/mtt+\sigma^2/2)^2/2\sigma^2}$$

is selected as a modelling function for the intravasal dye transporting function g(t), the parameters to be varied being formed by the mean transit time of the dye solution mtt and the relative dispersion $\sigma$.

6. The device of claim 4 and/or 5, wherein the modelling function for N compartments perfused at different strengths is modified by the sum of N logarithmic normal distributions of the form

$$g(t) = \Sigma\ \alpha_i\ g_{log\ nori}\ (t, mtt_i, \sigma_i)$$

with $\alpha_i > 0$ and with the secondary condition that $\Sigma\ \alpha_i = 1$.

7. The device of at least one of claims 1 to 6, wherein a blood flow index based on the organ dye transporting function g(t) is defined by the formula

$$\text{blood flow index} = \text{inflow maximum / inflow time}$$

and is directly proportional to the actual organ perfusion, the inflow time being established by the formula

$$\text{inflow time [s]} = t_{(y\%\ of\ signal\ build-up)} - t_{(x\%\ of\ signal\ build-up)}$$

t being the time[s] and x and y being variables defining the beginning and end of the tested inflow time interval of the transporting function.

8. The device of at least one of claims 1 to 7, **characterised by** a plurality of near-infrared spectroscopes connected to the computer for picking up cerebral dye curves simultaneously over various areas of the brain, for local measurement of the blood flow in various areas of the brain.

9. The device of at least one of claims 1 to 8, **characterised in that** local and/or deep resolution of the blood flow measurement is made possible by near-infrared spectroscopic measurement of the passage of an indicator dye with absorption properties in the near-infrared spectrum by means of a plurality of spatially-arranged light sensors.

10. The device of at least one of claims 1 to 9, wherein different dyes in differing quantities and concentrations are used as indicator dyes, and measurements by near-infrared spectroscopy are taken at different wavelengths.

11. The device of at least one of claims 1 to 10, **characterised in that** further circulatory parameters are quantified, i.e. the cardiac output according to the Stewart Hamilton equation, the intrathoracic blood volume as a product of the mean transit time (mtt) and the cardiac output and - also with local resolution - the relative blood volume as a product of the mean transit time (mtt) of the transporting function g(t) and the relative blood flow index of the corresponding region.

## Revendications

**1.** Agencement permettant la détermination non invasive du flux sanguin à travers un organe après application intraveineuse d'un colorant indicateur à propriétés d'absorption dans le spectre du proche infrarouge, **caractérisé par** un dispositif de mesure densitométrique à impulsions non invasif servant à la mesure séparée de la courbe de colorant artérielle a(t) dans le circuit systémique et deux spectroscopes en proche infrarouge devant être dirigés sur l'organe, ou davantage, servant à la détermination d'une courbe de colorant d'organe c(t), constitués chacun de deux sources de lumière monochromatique pulsée dans le domaine de longueurs d'onde du spectre du proche infrarouge, ou davantage, une longueur d'onde de mesure de ceux-ci correspondant le plus possible au maximum d'absorption du colorant indicateur utilisé, tandis qu'une longueur d'onde de référence comporte une longueur d'onde pour laquelle le colorant indicateur utilisé possède des propriétés d'absorption les plus faibles possible, ainsi que, pour chacun, un ou plusieurs capteurs de lumière associés servant de capteurs de mesure qui sont reliés par un câble de fibre de verre à un photomultiplicateur, en vue de la mesure séparée des courbes de colorant d'organe avec une résolution régionale.

**2.** Agencement suivant la revendication 1, **caractérisé en ce que** le dispositif de mesure densitométrique à impulsions et les spectroscopes de proche infrarouge sont reliés à un calculateur en vue de la détermination de la fonction de transport g(t) à l'aide de l'intégrale de convolution,

$$c(t) = \int_0^\infty g(t-u)\ a(u)\ du$$

u étant une variable auxiliaire de l'intégration, par convolution des courbes de colorant artérielles a(t) mesurées et de la courbe de colorant d'organe c(t) déterminée au moyen des spectroscopes de proche infrarouge.

**3.** Agencement suivant la revendication 2, dans lequel le calculateur détermine les fonctions de transport g(t) à partir de la courbe de colorant artérielle a(t) mesurée et de la courbe de colorant d'organe c(t), au moyen d'un procédé d'adaptation non linéaire, itératif, la variation de base de la fonction de transport étant décrite au moyen de fonctions types paramétrisées.

**4.** Agencement suivant la revendication 3, **caractérisé en ce que**, conformément à la méthode des plus petits carrés, les paramètres font l'objet d'une variation jusqu'à ce que soit rendue minimale l'erreur qui est déterminée par les carrés des différences

$$[c(t) - \int_0^\infty g(t-u)\ a(u)\ du]^2$$

et qui reproduit la différence entre la courbe de colorant d'organe c(t) mesurée d'une manière spectrométrique dans le proche infrarouge et le résultat de convolution de la courbe de colorant artérielle a(t) mesuré d'une manière densitométrique à impulsions.

**5.** Agencement suivant la revendication 4, dans lequel, en tant que fonction type pour la fonction de transport de colorant intravasculaire g(t), il est choisi une distribution de Gauss logarithmique de forme

$$g(t) = g_{lognor}(t, mtt, \sigma) = (1/\sqrt{2\pi}\ \sigma t)e^{-(\ln t/mtt + \sigma^2/2)^2/2\sigma^2}$$

les paramètres devant être soumis à une variation étant formés au moyen du temps de transit moyen de la solution de colorant mtt et de la dispersion relative σ.

**6.** Agencement suivant la revendication 4 et/ou 5, dans lequel la fonction type pour N compartiments pénétrés avec une intensité différente est modifiée au moyen de la somme de N distributions de Gauss logarithmiques de forme

$$g(t) = \Sigma \ \alpha_i g_{\log \text{nor}i}(t, mtt_i, \sigma_i)$$

avec $\alpha_i > 0$ et la condition secondaire $\Sigma \ \alpha_i = 1$.

**7.** Agencement suivant au moins l'une des revendications 1 à 6, dans lequel, à l'aide de la fonction de transport de colorant d'organe g(t), un indice de flux sanguin est déterminé conformément à la relation

indice de flux sanguin =

maximum d'application de flux/temps d'application de flux

lequel est directement proportionnel à la traversée sanguine effective d'organe, avec, pour la détermination du temps d'application de flux :

temps d'application de flux (s) =

$$t_{(y\% \text{ de l'accroissement de signal})} - t_{(x\% \text{ de l'accroissement de signal})},$$

t étant le temps (s) et x et y des variables qui définissent le début et la fin de l'intervalle d'application de flux de la fonction de transport, dans le temps, qui est examiné.

**8.** Agencement suivant au moins l'une des revendications 1 à 7, **caractérisé par** plusieurs spectroscopes de proche infrarouge reliés au calculateur et servant à la détection de courbes de colorant cérébrales, simultanément, au moyen de différentes aires du cerveau en vue de la mesure régionale du flux sanguin dans différentes aires du cerveau.

**9.** Agencement suivant au moins l'une des revendications 1 à 8, **caractérisé en ce qu'**au moyen d'une mesure spectroscopique de proche infrarouge du passage d'un colorant indicateur à propriétés d'absorption dans le spectre du proche infrarouge, à l'aide de plusieurs capteurs de lumière disposés dans l'espace à trois dimensions, une résolution régionale ou en profondeur de la mesure du flux sanguin est permise.

**10.** Agencement suivant au moins l'une des revendications 1 à 9, dans lequel différents colorants sont utilisés en des quantités et des concentrations différentes en tant que colorants indicateurs et des mesures spectroscopiques dans le proche infrarouge sont effectuées pour différentes longueurs d'onde.

**11.** Agencement suivant au moins l'une des revendications 1 à 10, **caractérisé en ce que** d'autres paramètres circulatoires, à savoir le débit cardiaque conforme à l'équation de Stewart-Hamilton, le volume sanguin intrathoracique en tant que produit du temps de transit moyen (mtt) et du débit cardiaque, ainsi que - également avec une résolution régionale - le volume sanguin relatif en tant que produit du temps de transit moyen (mtt) de la fonction de transport g(t) et de l'indice de flux sanguin relatif de la région correspondante, sont quantifiés.

EP 0 928 156 B1

## Figur 1A

**Sendeeinheiten**

**Empfängereinheiten**

Ablauf- A130
steuerung

monochromatische M100
Lichtquelle

monochromatische R100
Lichtquelle

monochromatische M101
Lichtquelle

monochromatische R101
Lichtquelle

Lichtaufnehmer L110

Lichtaufnehmer L210

Lichtaufnehmer L111

Photo- P120
multiplier

Photo- P220
multiplier

Photo- P121
multiplier

Signal- S130
aufbereitung

Aus- A140
werteeinheit

LED-Treiber 102

Sender 103

Ablauf- 112
steuerung

Detektor- 113
einheit

Ver- 123
stärker

PDD- 133
auswertung

# Abbildung 1B

# Figur 2

A — arterielle Farbstoffkurve.

B — zerebrale Farbstoffkurve (rechts)

C — zerebrale Farbstoffkurve (links)

Zeit [s]

# Figur 3